# EUROPEAN PATENT APPLICATION

(11) **EP 4 692 355 A1**
(43) Date of publication of application: **11.02.2026**
(21) Application number: 24784929.2
(22) Date of filing: 03.04.2024
(51) Int. Cl.: C12N 15/62, A61K 31/7088, A61K 48/00, A61P 25/00, C07K 14/705, C07K 19/00, C12N 5/10, C12N 15/864

(54) **NUCLEIC ACID CONSTRUCT CODING FOR INSULIN RECEPTOR FRAGMENT AND USE THEREOF**

(30) Priority: 05.04.2023 JP 2023061682
(71) Applicant: Tokyo Metropolitan Institute of Medical Science, Tokyo 156-8506 (JP)
(72) Inventor: HARADA, Takayuki, Tokyo 156-8506 (JP); NAMEKATA, Kazuhiko, Tokyo 156-8506 (JP)
(74) Representative: Graf von Stosch Patentanwaltsgesellschaft mbH
(86) International application number: PCT/JP2024/013706
(87) International publication number: WO 2024/210145

(57) **Abstract**

Provided are a novel nucleic acid construct encoding an insulin receptor fragment, use of the nucleic acid construct, etc. An aspect of the present invention is a nucleic acid construct encoding a fusion polypeptide which includes an intracellular domain of an insulin receptor and a membrane localization sequence.

## Description

### Technical Field

The present invention relates to a nucleic acid construct encoding an insulin receptor fragment and to use of the nucleic acid construct.

### Background Art

It is well known that insulin receptors are expressed in the liver, muscle, and adipose tissue, and function as metabolic regulators in the presence of insulin. It has further been revealed that insulin receptors are widely distributed even in the brain, and are involved also in the formation of the neural network, and high-order functions of the brain such as learning and memory (Non-Patent Literatures 1 to 3). In addition, reductions in insulin receptors and insulin, which lead to deposition of amyloid-beta protein (Aβ) and abnormal phosphorylation of tau protein, have been reported to be associated with cognitive function (Non-Patent Literatures 4 to 6).

There is also a report indicating that repeated administration of insulin was able to suppress nerve degeneration in model mice with optic nerve injury (Non-Patent Literature 7).

### Citation List

### [Non-patent Literature]

[Non-patent Literature 1]
   Bruning JC, Gautam D, Burks DJ, Gillette J, Schubert M, Orban PC, Klein R, Krone W, Muller-Wieland D, Kahn CR. Role of brain insulin receptor in control of body weight and reproduction. Science. 2000 Sep 22; 289(5487): 2122-5. doi: 10.1126/science.289.5487.2122. PMID: 11000114.
[Non-patent Literature 2]
   Schwartz MW, Woods SC, Porte D Jr, Seeley RJ, Baskin DG. Central nervous system control of food intake. Nature. 2000 Apr 6; 404(6778): 661-71. doi: 10.1038/35007534. PMID: 10766253.
[Non-patent Literature 3]
   Schubert M, Gautam D, Surjo D, Ueki K, Baudler S, Schubert D, Kondo T, Alber J, Galldiks N, Kustermann E, Arndt S, Jacobs AH, Krone W, Kahn CR, Bruning JC. Role for neuronal insulin resistance in neurodegenerative diseases. Proc Natl Acad Sci U S A. 2004 Mar 2; 101(9): 3100-5. doi: 10.1073/pnas.0308724101. Epub 2004 Feb 23. PMID: 14981233; PMCID: PMC365750.
[Non-patent Literature 4]
   Moroo I, Yamada T, Makino H, Tooyama I, McGeer PL, McGeer EG, Hirayama K. Loss of insulin receptor immunoreactivity from the substantia nigra pars compacta neurons in Parkinson's disease. Acta Neuropathol. 1994; 87(4): 343-8. doi: 10.1007/BF00313602. PMID: 8017169.
[Non-patent Literature 5]
   Gasparini L, Xu H. Potential roles of insulin and IGF-1 in Alzheimer's disease. Trends Neurosci. 2003 Aug; 26(8): 404-6. doi: 10.1016/S0166-2236(03)00163-2. PMID: 12900169.
[Non-patent Literature 6]
   Kellar D, Craft S. Brain insulin resistance in Alzheimer's disease and related disorders: mechanisms and therapeutic approaches. Lancet Neurol. 2020 Sep; 19(9): 758-766. doi: 10.1016/S1474-4422(20)30231-3. Epub 2020 Jul 27. PMID: 32730766; PMCID: PMC9661919.
[Non-patent Literature 7]
   Agostinone J, Alarcon-Martinez L, Gamlin C, Yu WQ, Wong ROL, Di Polo A. Insulin signalling promotes dendrite and synapse regeneration and restores circuit function after axonal injury. Brain. 2018 Jul 1; 141(7): 1963-1980. doi: 10.1093/brain/awy142. PMID: 29931057; PMCID: PMC6022605.

### Summary of Invention

### Technical Problem

As above, the relationship between insulin and the nervous system has been elucidated in various ways, and medical applications in the field of neurological disorders are being anticipated. However, in a case of applying, for example, the method disclosed in Non-Patent Literature 7 to humans, there are various problems. For example, it is more difficult to deliver insulin into eyes via eye drops in humans, and inherently, insulin administration is not sustainable. Nevertheless, repeated intraocular administration of insulin is impractical. In addition, in a case of repeated administration of insulin, there is the risk of side effects including hypoglycemia, depending on factors such as the administration frequency, dosage, and administration site.

The present invention has been made in view of the above problems, and an object thereof is to provide a novel nucleic acid construct encoding an insulin receptor fragment and use of the nucleic acid construct, as an alternative to, for example, insulin administration.

### Solution to Problem

In order to solve the above problems, the present invention includes the following aspects.
(i) A nucleic acid construct encoding a fusion polypeptide which includes an intracellular domain of an insulin receptor and a membrane localization sequence.
(ii) A cell activation method including the step of localizing, via a membrane localization sequence, an intracellular domain of an insulin receptor to a cell membrane within a cell.
(iii) A fusion polypeptide including an intracellular domain of insulin and a membrane localization sequence.

### Advantageous Effects of Invention

With an aspect of the present invention, it is possible to provide a novel nucleic acid construct encoding an insulin receptor fragment, and use of the nucleic acid construct.

### Brief Description of Drawings

Fig. 1 is a diagram illustrating the structure of a constitutively active F-iIR molecule expression vector.
Fig. 2 is diagram illustrating results regarding Example 1.
Fig. 3 is a diagram illustrating a method for a pathological analysis of an optic nerve crush model of Example 2 and results of the analysis.
Fig. 4 is a diagram illustrating a result of verification of dendrite regeneration of Example 3.
Fig. 5 is a diagram illustrating other results of the verification of the dendrite regeneration of Example 3.
Fig. 6 is a diagram illustrating an amino acid sequence (corresponding to SEQ ID NO: 6) of a fusion polypeptide used in Examples.

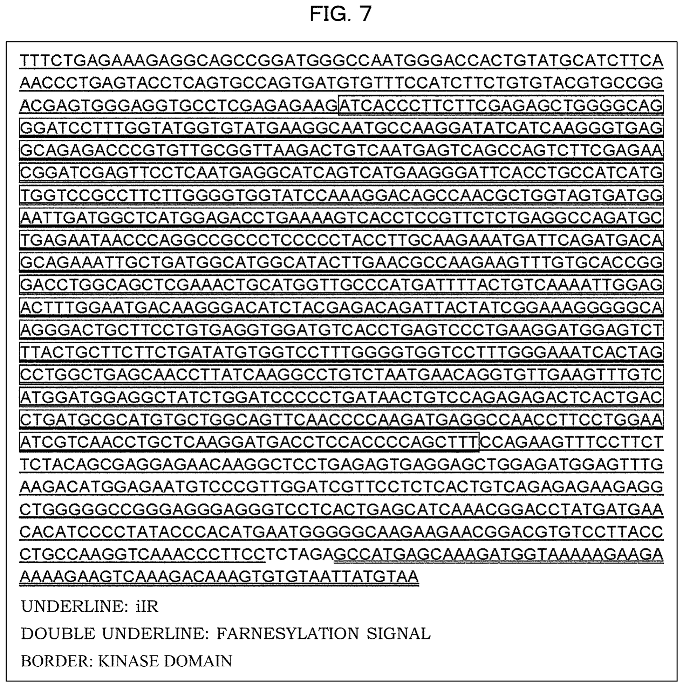

Fig. 7 is a diagram illustrating a base sequence (corresponding to SEQ ID NO: 3) of a gene that encodes the fusion polypeptide used in Examples.
Fig. 8 is a diagram illustrating a result of verification of retinal function recovery of Example 4.
Fig. 9 is a diagram illustrating a result of verification of visual function protection of Example 4.

### Description of Embodiments

### [Definitions of terms, etc.]

As used herein, the term "polynucleotide" is interchangeable with a "nucleic acid" or a "nucleic acid molecule". Unless otherwise specified, the term "polynucleotide" encompasses a polynucleotide which contains a known analog of a naturally-occurring nucleotide that is capable of functioning similarly to the naturally-occurring nucleotide. The term "base sequence" is interchangeable with a "nucleic acid sequence" or a "nucleotide sequence". Unless otherwise indicated, the term "base sequence" is intended to mean a deoxyribonucleotide sequence or a ribonucleotide sequence. The polynucleotide may be single-stranded, or may be double-stranded, and in the case of being single-stranded, the polynucleotide may be a sense strand, or may be an antisense strand.

As used herein, the term "gene" refers to a "polynucleotide" encoding a protein.

As used herein, the term "expression regulatory region" of a gene refers to a "polynucleotide" that regulates expression of the gene. Examples of the "expression regulatory region" include a promoter region and an enhancer region.

As used herein, the term "expression cassette" refers to an expression unit which includes (i) an expression regulatory region that is functional in an expression host and (ii) a polynucleotide that is operably linked to the expression regulatory region. In the expression cassette, the polynucleotide is preferably a gene or a gene fragment. An example of the expression cassette is an expression cassette in which the above expression regulatory region is linked to the above polynucleotide through genetic engineering. The term "operably linked" refers to a state in which expression of a polynucleotide is regulated via an expression regulatory sequence. The expression cassette may be in the form of an expression vector.

In the present specification, the term "polypeptide" is interchangeable with "protein". The "polypeptide" includes a structure of amino acids linked via a peptide bond. The "polypeptide" may further include, for example, a structure of a sugar chain, an isoprenoid group, or the like. Unless otherwise specified, the term "polypeptide" encompasses a polypeptide which contains a known analog of a naturally-occurring amino acid that is capable of functioning similarly to the naturally-occurring amino acid.

### [1. Nucleic acid construct]

A nucleic acid construct in accordance with an embodiment of the present invention is a nucleic acid construct encoding a fusion polypeptide which includes an intracellular domain of an insulin receptor and a membrane localization sequence. The present invention has been made based on the surprising knowledge that causing the nucleic acid construct to be expressed in a cell enables activation of an intracellular signaling pathway downstream of the insulin receptor without administration of insulin. That is, the present invention relates to a novel cell activation technique of localizing, via the membrane localization sequence of the fusion polypeptide encoded by this nucleic acid construct, the intracellular domain of an insulin receptor to a cell membrane.

The nucleic acid construct in accordance with the present embodiment provides, for example, effects typified by the following.
1) It is not necessary to administer insulin.
2) The molecular size required for activation can be considerably reduced in comparison with the entire length of the insulin receptor. This enables high-level expression of the molecules. Further, since the flexibility in designing a nucleic acid construct is considerably increased, it is possible, for example, to more easily achieve high-level expression, an improvement in gene introduction efficiency, etc.
3) Carcinogenicity and cytotoxicity are relatively low.

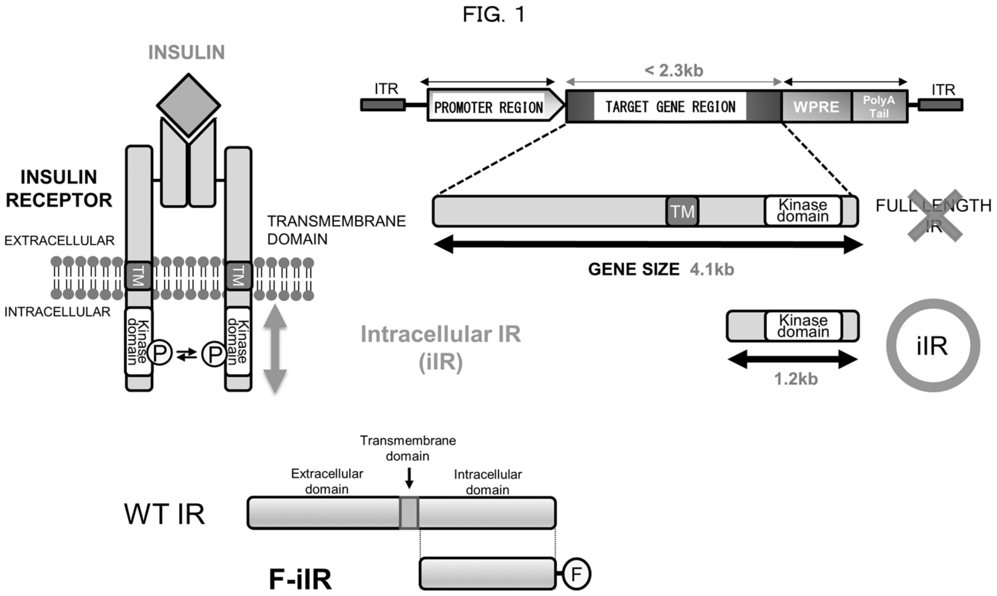

The insulin receptor belongs to the so-called receptor tyrosine kinase family, and is a heterotetramer composed of two α subunits, which bind insulin extracellularly, and two β subunits, which contain a trans-cell membrane domain and an intracellular domain that exhibits tyrosine kinase activity (see also Fig. 1). In general receptor tyrosine kinases, ligand reception causes dimerization of the molecules of the receptor, and the tyrosine kinases, which correspond to the intracellular domain, activate. In contrast, for the insulin receptor, the heterodimers, each consisting of an α subunit and a β subunit, are bound together by at least two disulfide bridges even before insulin reception. Upon insulin reception, a conformational change occurs in the insulin receptor, and the tyrosine kinases, which correspond to the intracellular domain, activate. One typical option that can be taken by those skilled in this technical field may, first of all, be gene therapy using a gene encoding the insulin receptor. However, due to a molecular weight-related matter, a gene vector encoding a full-length insulin receptor is subject to limitations regarding the size of a promoter used and any other factors. It is therefore difficult to induce high-level expression. Furthermore, since not only the insulin receptor is overexpressed but periodic administration of insulin is considered also necessary at the same time, gene therapy research utilizing an insulin receptor has not been put into practical use to date.

### (Fusion polypeptide)

The fusion polypeptide encoded by the nucleic acid construct in accordance with the present embodiment includes (1) an intracellular domain of an insulin receptor and (2) a membrane localization sequence, each of which will be described later. The membrane localization sequence can preferably be provided on the N-terminus side of the fusion polypeptide or on the C-terminus side of the fusion polypeptide, depending on the characteristics of the membrane localization sequence. In the relationship with the intracellular domain of the insulin receptor, the membrane localization sequence may be provided on the N-terminus side of the intracellular domain (part of the intracellular domain closer to the transmembrane domain) or may be provided on the C-terminus side of the intracellular domain. Note that in the constitutively active F-iIR molecules indicated in Examples, the membrane localization sequence is provided on the C-terminus side of the fusion polypeptide and provided on the C-terminus side of the intracellular domain of the insulin receptor. Thus, in the constitutively active F-iIR molecules indicated in the Examples, with respect to the cell membrane, the intracellular domain of the insulin receptor is located so as to be opposite to a typical position (see also Fig. 1).

In one example, the fusion polypeptide may include "another sequence" other than "the intracellular domain of an insulin receptor and the membrane localization sequence". Examples of the "another sequence" include a spacer sequence and an SH2 sequence of p85. The position in which the "another sequence" is inserted may be set according to the purpose. For example, the position can be between the intracellular domain of the insulin receptor and the membrane localization sequence, or on the N-terminus side of the fusion polypeptide (in this case, the membrane localization sequence can preferably be provided on the C-terminus side), or on the C-terminus side of the fusion polypeptide (in this case, the membrane localization sequence can preferably be provided on the N-terminus side). The "another sequence" may or may not include a sequence that dimerizes the fusion polypeptides of the present embodiment. In some cases, the "another sequence" preferably may not include such a sequence.

In a preferable example, the number of amino acids in the fusion polypeptide is in the range of not less than 280 and not more than 600, can more preferably be in the range of not less than 280 and not more than 500, and can particularly preferably be in the range of not less than 280 and not more than 450, 440, or 430. In a preferable example, the number of amino acids in the "intracellular domain of an insulin receptor and the membrane localization sequence" in the fusion polypeptide can preferably be in the range of not less than 280, not less than 290, not less than 300, or not less than 310 and not more than 450, not more than 440, or not more than 430. The fusion polypeptide having a small size has advantages such as, for example, (1) increased flexibility in designing other configurations in the nucleic acid construct (i.e., configurations other than the region encoding the fusion polypeptide) and (2) an improved expression efficiency of the fusion polypeptide. In particular, having a wider range of options for a promoter, etc. in the nucleic acid construct significantly contributes to improving the expression efficiency of the fusion polypeptide.

In one example, the fusion polypeptide may include a part of the extracellular domain of an insulin receptor and/or a part of the transmembrane domain of the insulin receptor. In a preferable example, the fusion polypeptide does not include the extracellular domain of an insulin receptor and/or the transmembrane domain of the insulin receptor. In a more preferable example, the fusion polypeptide includes neither the extracellular domain of an insulin receptor nor the transmembrane domain of the insulin receptor.
A specific example of the fusion polypeptide is a polypeptide represented by the amino acid sequence set forth in SEQ ID NO: 6, or a polypeptide represented by the amino acid sequence having not less than 90%, not less than 91%, not less than 92%, not less than 93%, not less than 94%, not less than 95%, not less than 96%, not less than 97%, not less than 98%, or not less than 99% sequence identity to the amino acid sequence set forth in SEQ ID NO: 6.

### (Intracellular domain of insulin receptor)

In the present embodiment, the intracellular domain of an insulin receptor refers to a polypeptide (i) corresponding to the intracellular domain of the β subunit of the insulin receptor, which is a single-span transmembrane protein, and a polypeptide (ii) functionally equivalent to the polypeptide (i). The intracellular domain is also referred to as a cytoplasmic domain.

The intracellular domain of an insulin receptor is only required to (1) have tyrosine kinase activity and/or (2) be capable of activating an intracellular signaling pathway downstream of the insulin receptor in a case of being localized to a cell membrane. The activation of the intracellular signaling pathway downstream of an insulin receptor can be ascertained by a known technique, e.g., ascertained as increased expression of at least one selected from Ras, ERK1/2, PI3K, Akt, phospholipase C-y, etc.

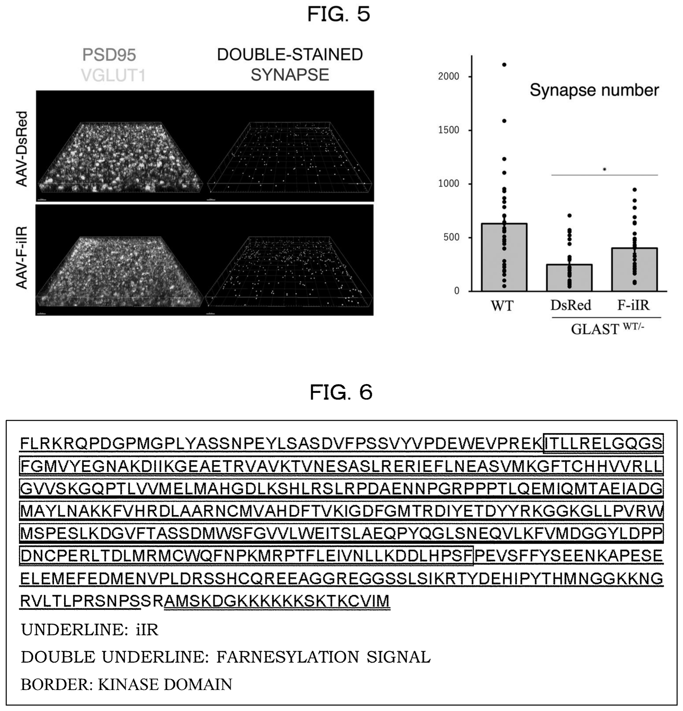

The intracellular domain of an insulin receptor is not particularly limited, but examples thereof preferably include the following.
1) A polypeptide represented by the amino acid sequence set forth in SEQ ID NO: 5. SEQ ID NO: 5 is an example of the amino acid sequence of the intracellular domain of a mouse-derived insulin receptor.
2) A polypeptide functionally equivalent to the polypeptide represented by the amino acid sequence set forth in SEQ ID NO: 5. Examples of such a functionally equivalent polypeptide include the following.
2-a) A partial sequence of the polypeptide represented by the amino acid sequence set forth in SEQ ID NO: 5, the partial sequence containing at least a kinase domain (276 amino acids in length). An amino acid sequence of the amino acid sequence set forth in SEQ ID NO: 5 that corresponds to the kinase domain is indicated by a border in Fig. 6.
2-b) A polypeptide derived from a mammal (e.g., human) other than a mouse, the polypeptide corresponding to either 1) or 2-a). For example, the amino acid sequence of the intracellular domain of a human-derived insulin receptor is as set forth in SEQ ID NO: 7. A partial sequence of the polypeptide represented by the amino acid sequence set forth in SEQ ID NO: 7, the partial sequence containing at least a kinase domain, is also one of the examples. The kinase domain of the polypeptide represented by the amino acid sequence set forth in SEQ ID NO: 7 can easily be ascertained by comparison with the amino acid sequence set forth in SEQ ID NO: 5.
2-c) A polypeptide represented by an amino acid sequence having not less than 90% sequence identity to the amino acid sequence of the polypeptide described in 1), 2-a), or 2-b). The sequence identity is more preferably not less than 91%, not less than 92%, not less than 93%, not less than 94%, not less than 95%, not less than 96%, not less than 97%, not less than 98%, or not less than 99%.

### (Membrane localization sequence)

The membrane localization sequence is a peptide sequence for localizing the above fusion polypeptide to a cell membrane. The membrane localization sequence preferably induces lipid modification of an amino acid. The lipid modification of an amino acid is not limited to a particular type, but examples thereof include (1) fatty-acylation, (2) prenylation, (3) glycosylphosphatidylinositol anchoring, and (4) cholesterolation, and more specific examples thereof include myristoylation, palmitoylation, farnesylation, and geranylgeranylation. In particular, said lipid modification is preferably farnesylation or geranylgeranylation.

Specific examples of an amino acid sequence motif forming the membrane localization sequence include the following: (1) Met-Gly-X-X-X-Ser-X-X-X (N-terminus-side lipid modification motif (N-myristoylation)); and (2) a CAAX motif, a CC motif, a CXC motif, a CCXX motif (C-terminus-side lipid modification motif), where X refers to any amino acid, A refers to an aliphatic amino acid (e.g., isoleucine, leucine, valine, alanine, methionine), and C refers to cysteine.

### (Aspect of nucleic acid construct)

An aspect of the nucleic acid construct in accordance with the present embodiment is a gene encoding the above fusion polypeptide which includes the intracellular domain of an insulin receptor and a membrane localization sequence. An example of the gene is a gene having a base sequence set forth in SEQ ID NO: 3, or a gene having a base sequence having not less than 85%, not less than 90%, not less than 91%, not less than 92%, not less than 93%, not less than 94%, not less than 95%, not less than 96%, not less than 97%, not less than 98%, not less than 99% sequence identity to the base sequence set forth in SEQ ID NO: 3. An example of a gene encoding the intracellular domain of an insulin receptor included in the above fusion polypeptide is a gene having a base sequence set forth in SEQ ID NO: 2, or a gene having a base sequence having not less than 85%, not less than 90%, not less than 91%, not less than 92%, not less than 93%, not less than 94%, not less than 95%, not less than 96%, not less than 97%, not less than 98%, not less than 99% sequence identity to any of the base sequence set forth in SEQ ID NO: 2.

Another aspect of the nucleic acid construct in accordance with the present embodiment is an expression cassette which causes the above gene to be expressed. The expression cassette is preferably an expression vector, and more preferably a virus vector such as a retrovirus vector, a lentivirus vector, an adenovirus vector, an adeno-associated virus vector, a herpesvirus vector, and a vaccinia virus vector. Among these virus vectors, from the viewpoint of use in gene therapy of neurological disorders, an adeno-associated virus vector is preferable. In particular, from the viewpoint of use in gene therapy of neurological disorders, an adeno-associated virus vector such as a serotype 1 (AAV1) which exhibits tissue tropism toward the central nervous system, etc., a serotype 2 (AAV2) which exhibits broad tissue tropism, and a serotype 5 (AAV5) which exhibits tissue tropism toward the central nervous system, retina, etc. can be preferable.

The above expression cassette includes any expression regulatory region that is functional in an expression host. A promoter serving as the expression regulatory region is not limited to a particular type, but specific examples thereof can preferably include a promoter that provides high-level expression in mammals (in particular, human) or selectively functions in a specific tissue, such as a CAG promoter, a CMV promoter, or SYN (synapsin) I. In particular, the promoter can preferably be the CAG promoter. In the expression cassette, the promoter is provided on the upstream side of the gene.

The above expression cassette may further include, as necessary, a functional sequence that has an effect of, e.g., increasing the stability of mRNA produced through transcription, and that functions to, e.g., improve gene expression. Examples of the functional sequence include a woodchuck hepatitis virus posttranscriptional regulatory element (WPRE) sequence. In the expression cassette, the WPRE sequence is provided on the downstream side of the gene.

Another example of the functional sequence that may be included in the above expression cassette is a poly-A tail addition signal sequence. Examples of the poly-A tail addition signal sequence include the poly-A tail addition signal sequence of simian virus 40 and the poly-A tail addition signal sequence of human growth hormone. The poly-A tail addition signal sequence of human growth hormone can be more preferable. In the expression cassette, the WPRE sequence is provided on the downstream side of the gene.

The above expression cassette may further include inverted terminal repeat sequences (ITRs) and the like. The ITRs are provided in the expression cassette so as to sandwich the entire transcription unit (from the promoter to the poly-A tail addition signal sequence) therebetween.

### [2. Pharmaceutical composition]

A pharmaceutical composition in accordance with an embodiment of the present invention is a gene therapeutic drug containing the above nucleic acid construct. In one example, the pharmaceutical composition is a drug for neurological disorders. As a more specific example, the pharmaceutical composition is for neuroprotection or neuroregeneration. This pharmaceutical composition exhibits at least one of the powerful effects of neuroprotection, axon regeneration, and dendrite regeneration (synapse regeneration effect), and preferably exhibits all of these effects. Thus, the pharmaceutical composition can be used for therapy and/or prevention of neurological disorders. The scope of the present invention encompasses a method for therapy and/or prevention of neurological disorders, the method including administering an effective amount of the pharmaceutical composition to an administration target.

### (Administration target)

A human or non-human animal as an administration target is preferably any one selected from the group consisting of mammals including human. The mammal as the administration target is not limited to a particular type, but examples thereof include laboratory animals such as mice, rats, rabbits, guinea pigs, and primates other than human; pet animals such as dogs and cats; farm animals such as cows and horses; and human, and said mammal is particularly preferably human. The human or non-human animal as the administration target has, for example, a neurological disorder described below.

### (Neurological disorder)

The neurological disorder to be treated and/or prevented is not limited to a particular type, but refers to a pathological condition in which a function of a neural cell is impaired due to denaturation of the neural cell and/or death of the neural cell. The causes of the denaturation and death of the neural cell are not particularly limited, but include damage to the nervous system resulting from an accident or the like.

Examples of the neurological disorder of the visual nervous system include, in addition to neurodegenerative diseases such as glaucoma, retinitis pigmentosa, age-related macular degeneration, and diabetic retinopathy, damage to the visual nervous system resulting from an accident or the like. In particular, a disorder accompanied by degeneration of retinal ganglion cells typified by glaucoma and damage to the visual nervous system (optic nerve injury) can be preferable as disorders to which the present invention is applied. Examples of the neurological disorder of the auditory nervous system include, in addition to a sensory nerve degenerative disease such as neural hearing loss, damage to the auditory nervous system resulting from an accident or the like. The insulin receptor has been revealed to be involved in high-order functions of the brain, and furthermore, decreases in insulin receptors and insulin have been revealed to lead to the deposition of amyloid-beta protein (Aβ) and the abnormal phosphorylation of tau protein. Thus, the improvement of cognitive functions impaired by dementia and any other condition can also preferably be a disorder to which the present invention is applied.

As indicated in the Examples described below, neuroprotection and an axon and dendrite regeneration effect were confirmed in disease-model mice with optic nerve injury or glaucoma. Furthermore, in a case where the retracted dendrites regenerated, the previously degenerated synapse formation was restored. This suggests the possibility that in cases where the cell bodies remain viable in neural tissue, synaptic reconnection will restore normal function.

### (Administration method, dosage, number of administrations, etc.)

A method of administering the pharmaceutical composition is not particularly limited. The pharmaceutical composition may be administered locally by a method such as injection (with use of a syringe, an injection pump, or the like), instillation into an eye, transdermal administration, or sublingual administration, or may be administered systemically by a method such as oral administration, intravascular administration such as intravenous administration or intraarterial administration, or enteral administration. In a preferable aspect of administration, the pharmaceutical composition is administered locally, in the vicinity of the nervous system targeted for therapy, etc.

The dosage (effective amount) of the pharmaceutical composition may be set as appropriate according to the age and sex of the human or animal as the administration target, the symptom, the administration route, the number of administrations, and the like. As necessary, an in vivo assay using the pharmaceutical composition can be carried out in advance to determine the dosage without requiring excessive experimentation.

The number of administrations of the pharmaceutical composition is not particularly limited provided that an effect is obtained, and can be set as appropriate, for example, according to the dosage, the administration route, the symptom, the age and sex of the human or animal.

### (Component other than nucleic acid construct)

A pharmaceutical composition in accordance with an embodiment of the present invention can include at least the above nucleic acid construct and a pharmaceutically acceptable carrier. The pharmaceutically acceptable carrier is not particularly limited, but preferably possesses the property of substantially not interfering with the functions of the nucleic acid construct and the property of not causing any substantial adverse effects on the human or non-human animal as an administration target. The pharmaceutical composition can preferably contain a liquid carrier such as water. As an example, the pharmaceutical composition may be a liposomal preparation.

Examples of the component of the above pharmaceutical composition further include, but are not particularly limited to, lubricants, preservatives, stabilizers, wetting agents, emulsifiers, osmotic pressure regulating salts, buffer agents, coloring agents, antioxidants, and viscosity modifiers.

### [3. Cell activation method, etc.]

A cell activation method in accordance with an embodiment of the present invention is a method including the step (localization step) of localizing, via a membrane localization sequence, an intracellular domain of an insulin receptor to a cell membrane within a cell. Typically, the localization step can be carried out by causing one selected from the group consisting of: the nucleic acid construct described in the section [1. Nucleic acid construct]; and the pharmaceutical composition described in the section [2. Pharmaceutical composition] to be expressed within a neural cell. The neural cell is not limited to a particular type, but examples of the neural cell include a cell of the central nervous system, a cell of the peripheral nervous system, and a precursor cell of any of these cells. The neural cell may be an isolated neural cell, or may be a neural cell present in the body of a human or non-human animal.

According to the cell activation method, it is possible to activate an intracellular signaling pathway downstream of an insulin receptor without administration of insulin. As a result of the cell activation, at least one of the powerful effects of neuroprotection, axon regeneration, and dendrite regeneration (synapse regeneration) is exhibited, and all of these effects are preferably exhibited. Thus, the cell activation method can be used for therapy and/or prevention of neurological disorders.

An embodiment of the present invention further provides a neural cell into which a nucleic acid construct described in the section [1. Nucleic acid construct] has been introduced to be capable of being expressed, a non-human animal having the neural cell, etc.

### [4. Main points]

As described above, the present invention includes the following aspects.
(1) A nucleic acid construct encoding a fusion polypeptide, the fusion polypeptide including: an intracellular domain of an insulin receptor; and a membrane localization sequence.
(2) The nucleic acid construct described in (1), in which the intracellular domain of the insulin receptor is selected from the following:
   1) a polypeptide represented by an amino acid sequence set forth in SEQ ID NO: 5; and
   2) a polypeptide functionally equivalent to the polypeptide represented by the amino acid sequence set forth in SEQ ID NO: 5.
(3) The nucleic acid construct described in (1) or (2), in which the membrane localization sequence induces lipid modification of amino acids.
(4) The nucleic acid construct described in (3), in which the membrane localization sequence is a farnesylation sequence.
(5) The nucleic acid construct described in (2), in which the intracellular domain of the insulin receptor is the polypeptide functionally equivalent to the polypeptide represented by the amino acid sequence set forth in SEQ ID NO: 5 and which is selected from the following polypeptides 1) to 3):
   1) a polypeptide represented by an amino acid sequence having not less than 90% sequence identity to the amino acid sequence set forth in SEQ ID NO: 5;
   2) a polypeptide represented by an amino acid sequence set forth in SEQ ID NO: 7; and
   3) a polypeptide represented by an amino acid sequence having not less than 90% sequence identity to the amino acid sequence set forth in SEQ ID NO: 7.
(6) The nucleic acid construct described in any one of (1) to (5), in which the nucleic acid construct is a vector.
(7) The nucleic acid construct described in (6), in which the nucleic acid construct is an adeno-associated virus vector.
(8) A pharmaceutical composition, including the nucleic acid construct according to any one of (1) to (7).
(9) The pharmaceutical composition described in (8), in which the pharmaceutical composition is a drug for neurological disorders.
(10) The pharmaceutical composition described in (9), in which the pharmaceutical composition is for neuroprotection or neuroregeneration.
(11) A neural cell into which the nucleic acid construct described in any one of (1) to (7) has been introduced to be capable of being expressed.
(12) A cell activation method, including the step of localizing, via a membrane localization sequence, an intracellular domain of an insulin receptor to a cell membrane within a cell.
(13) The cell activation method described in (12), in which the step is carried out by causing one selected from the group consisting of: the nucleic acid construct described in any one of (1) to (7); and the pharmaceutical composition described in any one of (8) to (10) to be expressed within a neural cell.
(14) A fusion polypeptide including an intracellular domain of insulin and a membrane localization sequence. This fusion polypeptide may be encoded by the nucleic acid construct described in any one of (1) to (7).

In the following, the embodiments of the present invention are described in more detail by indicating Examples. As a matter of course, the present invention is not limited to the Examples below and the details of the present invention can have various aspects. Further, the present invention is not limited to the embodiments, but can be altered by a skilled person in the art within the scope of the claims. Any embodiment derived by appropriately combining technical means disclosed in differing embodiments is within the scope of the present invention. All literatures described in this specification are incorporated herein by reference.

### Examples

### [Example 1] Preparation of constitutively active F-iIR molecule expression vector

### (Method)

An AAV vector for the expression of the intracellular domain of an insulin receptor insulin receptor molecule (intracellular insulin receptor; iIR) was prepared in the following manner. As the AAV vector into which to incorporate a gene fragment encoding the iIR, the pAAV-CAG-shuttle-WPRE manufactured by Applied Viromics was used. As the gene fragment F-iIR to be incorporated into the AAV vector, a DNA fragment that encodes a protein which has a myc-tag added to the N-terminus of the iIR and has a farnesylation signal sequence added to the C-terminus of the iIR was prepared (see also Fig. 1). That is, this gene fragment F-iIR was constructed by using only the intracellular domain of an insulin receptor without including the extracellular domain of the insulin receptor and transmembrane domain, and adding a myc-tag and a farnesylation signal sequence to the intracellular domain.

The farnesylation signal sequence (the base sequence and the amino acid sequence thereof are set forth in SEQ ID NO: 1 and SEQ ID NO: 4, respectively) is a signal sequence for localizing the protein to the membrane. The base sequence encoding the iIR is set forth in SEQ ID NO: 2, and the amino acid sequence of the iIR is set forth in SEQ ID NO: 5. The full-length base sequence of the gene fragment inserted in the AAV vector is set forth in SEQ ID NO: 3, and the amino acid sequence encoded by the gene fragment is set forth in SEQ ID NO: 6.

The obtained AAV vector (referred to as AAV-F-iIR) was designed to achieve high-level expression by using a CAG promoter as the expression control system for the gene fragment encoding the iIR, and further fusing a WPRE sequence with a poly-A sequence derived from human growth hormone (Human Growth Hormone). In this AAV vector, the WPRE sequence and the poly-A sequence derived from human growth hormone are provided downstream of the farnesylation signal sequence. The CAG promoter, the WPRE sequence, and the poly-A sequence derived from human growth hormone are originally incorporated into the parental AAV vector.

Subsequently, the obtained AAV-F-iIR was transfected into Cos7 cells, and the expression level and activity of the AAV-F-iIR were evaluated.

### (Results)

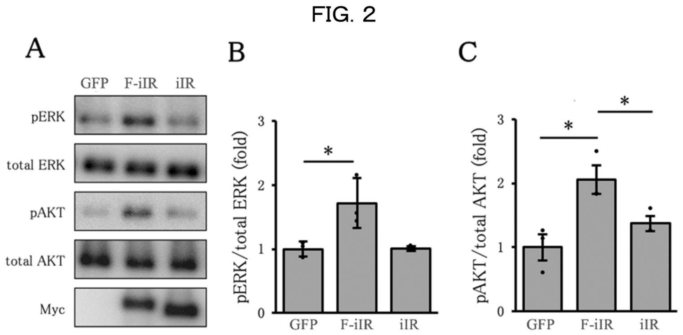

The results of the transfection of the AAV vector into the Cos7 cells are shown in Fig. 2. In Fig. 2, "F-iIR" refers to the AAV-F-iIR, "GFP" refers to the AAV vector (referred to as AAV-GFP) which has a gene fragment for a fluorescent protein GFP incorporated therein in place of the gene fragment F-iIR, and "iIR" refers to an AAV vector which has the gene fragment (SEQ ID NO: 2) of the iIR incorporated therein in place of the gene fragment F-iIR. Illustrated in A of Fig. 2 are the results of Western blot performed by transfecting each AAV vector into Cos7 cells, culturing the cells for 24 hours, and then lysing the cells, and with use of the anti-myc antibody (c-Myc antibody (9E10), Santa Cruz), the anti-pERK antibody (Phospho-p44/42 (Erk1/2) (Thr202/Thr204), Cell Signaling), the anti-pAKT antibody (Phospho-Akt (Ser473) (587F11), Cell Signaling), the anti-ERK antibody (p44/42 MAPK (Erk1/2) (137F5), Cell Signaling), and the anti-AKT antibody (Akt antibody, Cell Signaling).
In the Cos7 cells which expressed the F-iIR, pERK and pAKT were increased, and both of the signals were therefore confirmed to be activated (in particular, B and C of Fig. 2). However, there was no change in the total levels of ERK and AKT.

### [Example 2] Pathological analysis of optic nerve crush model

### (Method)

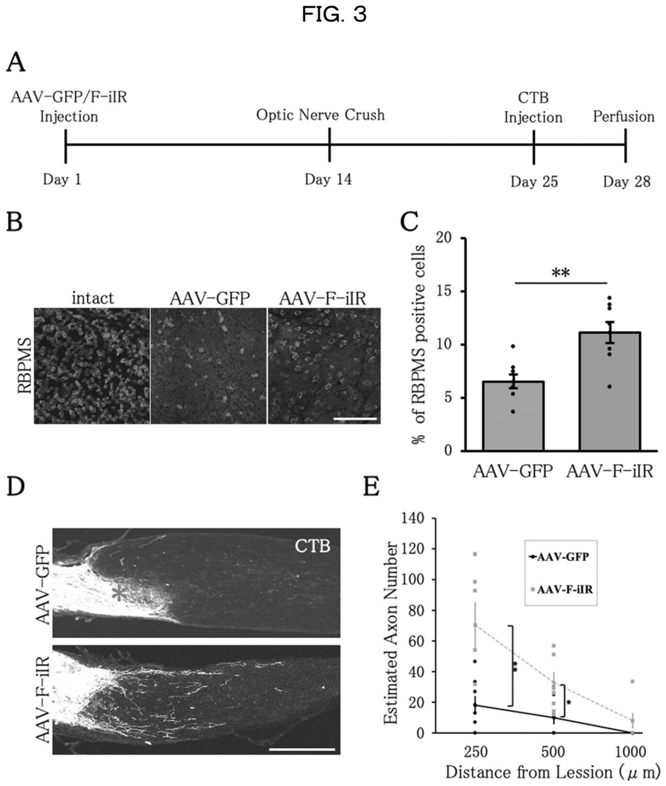

The neuroprotection effect was confirmed by performing tissue immunostaining with use of an antibody that recognizes RBPMS, which is a marker for retinal ganglion cells. Specifically, as illustrated in A of Fig. 3, mice (strain: C57BL/6, available from Charles River) were intraocularly administered 2 µL of the AAV-GFP (control group) or the AAV-F-iIR 14 days prior to optic nerve crush. Subsequently, optic nerves of the mice were exposed and physically crushed at a location approximately 1 mm posterior to the eyeball (for details of the method, see Non-Patent Literatures 10 and 11). Fourteen days after the optic nerve crush, retinal flat-mount specimens were prepared, and the number of surviving retinal ganglion cells was quantitatively counted.

Regarding optic nerve regeneration, as illustrated in A of Fig. 3, the same individuals used for observing the neuroprotection effect were used. Three days prior to optic nerve extraction, cholera toxin β-subunit (CTB), which was a fluorescent dye, was injected through the eyeballs to stain the regenerating axonal fibers (see also Non-Patent Literatures 12 and 13). Fourteen days before optic nerve crush, 2 µL of AAV-GFP administration (control group) or AAV-F-iIR was intraocularly administered. Fourteen days after the optic nerve crush, the mice were perfusion-fixed, and pathological specimens of the optic nerves were prepared, and the amount of axon regeneration from the crush site was quantified.

### (Results)

Regarding the neuroprotection effect, the number of surviving retinal ganglion cells in the AAV-F-iIR-injected group was significantly increased compared with the AAV-GFP-injected group (see B and C of Fig. 3). Regarding the optic nerve regeneration, the number of regenerating axons and the extension distance were significantly increased in the AAV-F-iIR-injected group compared with the control group (AAV-GFP) (see D and E of Fig. 3). In the graphs illustrated in Fig. 3, the X-axis represents the distance (µm) from the optic nerve crush site, and the Y-axis represents the number of axons. The symbol ** indicates p < 0.01, and the symbol * represents p < 0.05 (one-tailed Student's t-test).

### [Example 3] Verification of dendrite regeneration

### (Method)

Regarding dendrite regeneration, GLAST ^{WT/-} mice, which are a glaucoma model animal, were utilized (see Non-Patent Literatures 14 and 15). To visualize and measure dendrites, crossed mice (hereinafter referred to as "GLAST ^{WT/-}crossed mice") were used, which were generated by crossing GLAST ^{WT/-} mice with Thy1-GFP mice. In these crossed mice, dendrites degenerate in almost all retinal ganglion cells by 12 weeks of age. Therefore, in the GLAST ^{WT/-} crossed mice at 12 weeks of age, AAV-DsRed (control group) or the AAV-F-iIR was intraocularly administered, and the regeneration effect on dendrites were evaluated at 16 weeks of age. The AAV-DsRed is an AAV vector which has the gene fragment of a fluorescent protein DsRed incorporated therein in place of the gene fragment F-iIR.

On the other hand, regarding the observation of synapses, retinal tissue immunostaining was performed using respective antibodies against the postsynaptic marker (PSD95) and the presynaptic marker (GLUT1), and the number of double-stained cells labeled with these antibodies was quantified through automatic counting, via the image analysis software Imaris (see also the disclosures in Non-Patent Literature 13).

### (Results)

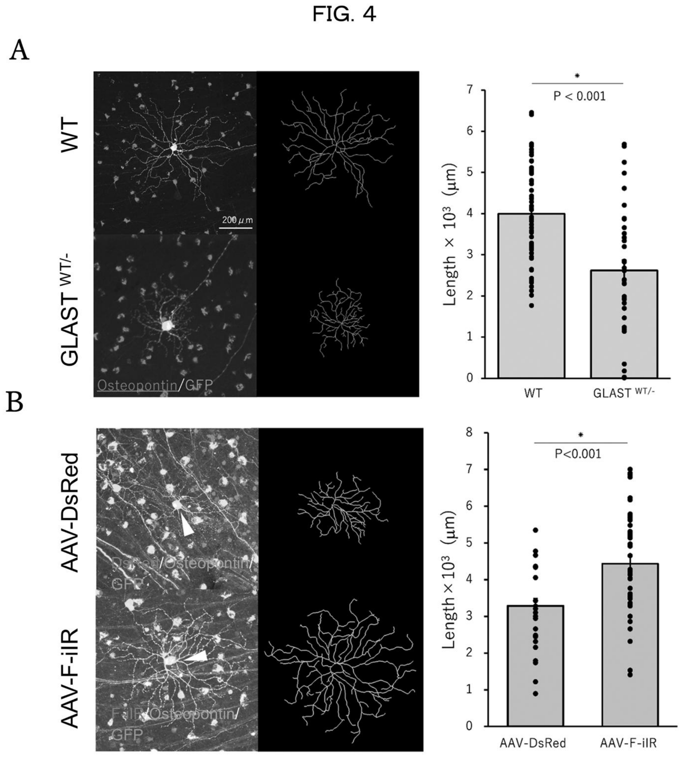

Using GLAST ^{WT/-} crossed mice, as a result of the measurement of the dendrite length of the retinal ganglion cells, it was observed that the dendrite lengths of almost all of the retinal ganglion cells remarkably shortened in 12-week-old GLAST ^{WT/-} crossed mice, compared with the Thy1-GFP mice (hereinafter referred to as wild-type mice) (A of Fig. 4). Next, when the AAV-DsRed was intraocularly administered to the 12-week-old GLAST ^{WT/-} crossed mice, and observation was carried out at 16 weeks of age, no change in the dendrite length was observed. On the other hand, in the AAV-F-iIR-injected group, the dendrite length significantly increased, and morphological recovery was also observed (B of Fig. 4). In Fig. 4, the symbol * indicates p < 0.001 (one-tailed Student's t-test). Regarding the results illustrated in Fig. 4, aRGCs, which are a subtype of retinal ganglion cells, were subjected to the observation of the dendrites. In A of Fig. 4, Osteopontin (αRGC marker) is indicates in red, and the GFP is indicated in green. In B of Fig. 4, the DsRed (upper row) or the F-iIR (lower row) are indicated in red, the Osteopontin is indicated in blue, and the GFP is indicated in green.

Similarly, the number of synapses in the retinal ganglion cells (RGCs) was measured in the tissue immunostaining method in which antibodies against the postsynaptic marker (PSD95) and the presynaptic marker (GLUT1) were used. When the AAV-DsRed was intraocularly administered to 12-week-old GLAST ^{WT/-} crossed mice and observation was carried out at 16 weeks of age, the number of synapses was greatly reduced compared with the wild-type mice (Fig. 5). On the other hand, in the AAV-F-iIR-injected group, a significant improvement in the number of synapses was observed as well, compared with the AAV-DsRed-injected group. In Fig. 5, the symbol * indicates p < 0.05 (one-tailed Student's t-test).

### [Example 4] Verification of recovery of retinal function and protection of visual function

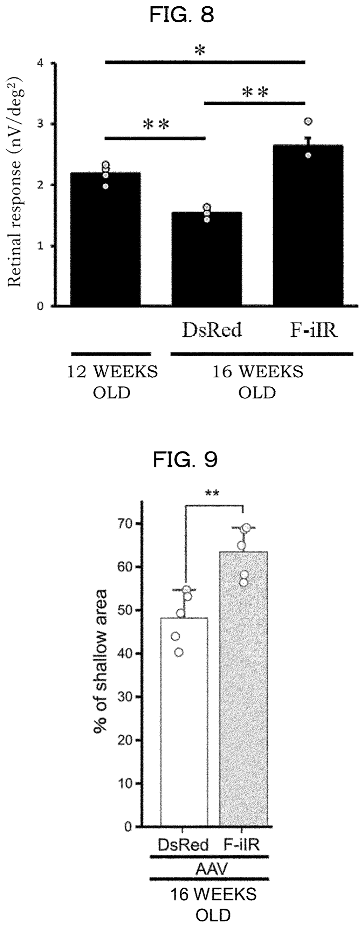

The electrophysiological response of the retina to visual input was evaluated by measuring multifocal electroretinograms with use of the visual evoked response imaging system (VERIS; product name). When the AAV-DsRed was intraocularly administered to the 12-week-old GLAST ^{WT/-}mice (glaucoma model mice) and observation was carried out at 16 weeks of age, retinal potentials were significantly reduced (Fig. 8). On the other hand, in the AAV-F-iIR-injected group, the retinal potentials were significantly increased compared with the AAV-DsRed-injected group, and a significant improvement was observed, even compared with the 12-week-old GLAST ^{WT/-} mice. In Fig. 8, the symbol * indicates p < 0.05, and the symbol ** indicates p < 0.01 (Tukey's multiple comparison test).

Furthermore, the visual function was evaluated using the Visual Cliff test (see Non-Patent Literature 16). When the AAV-DsRed or the AAV-F-iIR was intraocularly administered to the 12-week-old GLAST ^{WT/-} mice (glaucoma model mice) and observation was carried out at 16 weeks of age, it was observed that the visual function was significantly protected (the proportion of time spent in the shallow area increased) in the AAV-F-iIR-injected group compared with the AAV-DsRed-injected group (Fig. 9). In Fig. 9, the symbol ** indicates p < 0.01 (one-tailed Student's t-test).

### <List of reference literatures in Examples>

Non-Patent Literature 10:
   Namekata K, Harada C, Taya C, Guo X, Kimura H, Parada LF, Harada T. Dock3 induces axonal outgrowth by stimulating membrane recruitment of the WAVE complex. Proc Natl Acad Sci U S A 107(16) 7586-7591, 2010.
Non-Patent Literature 11:
   Namekata K, Harada C, Guo X, Kimura A, Kittaka D, Watanabe H, Harada T. Dock3 stimulates axonal outgrowth via GSK-3β-mediated microtubule assembly. J Neurosci 32(1): 264-274, 2012.
Non-Patent Literature 12:
   Nishijima E, Namekata K, Kimura A, Guo X, Harada C, Noro T, Nakano T, Harada T. Topical ripasudil stimulates neuroprotection and axon regeneration in adult mice following optic nerve injury. Sci Rep. 2020 Sep 24; 10(1): 15709. doi: 10.1038/s41598-020-72748-3. PMID: 32973242; PMCID: PMC7515881.
Non-Patent Literature 13:
   Nishijima E, Honda S, Kitamura Y, Namekata K, Kimura A, Guo X, Azuchi Y, Harada C, Murakami A, Matsuda A, Nakano T, Parada LF, Harada T. Vision protection and robust axon regeneration in glaucoma models by membrane-associated Trk receptors. Mol Ther. 2023 Mar 1; 31(3): 810-824. doi: 10.1016/j.ymthe.2022.11.018. Epub 2022 Dec 5. PMID: 36463402.
Non-Patent Literature 14:
   Harada T, Harada C, Nakamura K, Quah HM, Okumura A, Namekata K, Saeki T, Aihara M, Yoshida H, Mitani A, Tanaka K. The potential role of glutamate transporters in the pathogenesis of normal tension glaucoma. J Clin Invest. 2007 Jul; 117(7): 1763-70. doi: 10.1172/JCI30178. PMID: 17607354; PMCID: PMC1890997.
Non-Patent Literature 15:
   Sano H, Namekata K, Kimura A, Shitara H, Guo X, Harada C, Mitamura Y, Harada T. Differential effects of N-acetylcysteine on retinal degeneration in two mouse models of normal tension glaucoma. Cell Death Dis. 2019 Jan 28; 10(2): 75. doi: 10.1038/s41419-019-1365-z. PMID: 30692515; PMCID: PMC6349904.
Non-Patent Literature 16:
   Gibson, E. J., and Walk, R, D. The "Visual Cliff". Sci Am. 1960, Apr: 202: 64-71.

### Industrial Applicability

The present invention is applicable to medical uses targeting, for example, neurological disorders.

## Claims

1. A nucleic acid construct encoding a fusion polypeptide, the fusion polypeptide including: an intracellular domain of an insulin receptor; and a membrane localization sequence.

2. The nucleic acid construct according to claim 1, wherein
the intracellular domain of the insulin receptor is selected from the following:
1) a polypeptide represented by an amino acid sequence set forth in SEQ ID NO: 5; and
2) a polypeptide functionally equivalent to the polypeptide represented by the amino acid sequence set forth in SEQ ID NO: 5.

3. The nucleic acid construct according to claim 1 or 2, wherein
the membrane localization sequence induces lipid modification of amino acids.

4. The nucleic acid construct according to claim 3, wherein
the membrane localization sequence is a farnesylation sequence.

5. The nucleic acid construct according to claim 2, wherein
the intracellular domain of the insulin receptor is the polypeptide functionally equivalent to the polypeptide represented by the amino acid sequence set forth in SEQ ID NO: 5 and which is selected from the following polypeptides 1) to 3):
1) a polypeptide represented by an amino acid sequence having not less than 90% sequence identity to the amino acid sequence set forth in SEQ ID NO: 5;
2) a polypeptide represented by an amino acid sequence set forth in SEQ ID NO: 7; and
3) a polypeptide represented by an amino acid sequence having not less than 90% sequence identity to the amino acid sequence set forth in SEQ ID NO: 7.

6. The nucleic acid construct according to any one of claims 1 to 5, wherein
the nucleic acid construct is a vector.

7. The nucleic acid construct according to claim 6, wherein
the nucleic acid construct is an adeno-associated virus vector.

8. A pharmaceutical composition, comprising
the nucleic acid construct according to any one of claims 1 to 7.

9. The pharmaceutical composition according to claim 8, wherein
the pharmaceutical composition is a drug for neurological disorders.

10. The pharmaceutical composition according to claim 9, wherein
the pharmaceutical composition is for neuroprotection or neuroregeneration.

11. A neural cell into which the nucleic acid construct according to any one of claims 1 to 7 has been introduced to be capable of being expressed.

12. A cell activation method, comprising
the step of localizing, via a membrane localization sequence, an intracellular domain of an insulin receptor to a cell membrane within a cell.

13. The cell activation method according to claim 12, wherein
the step is carried out by causing the nucleic acid construct according to any one of claims 1 to 7 to be expressed within a neural cell.

14. A fusion polypeptide, comprising: an intracellular domain of insulin; and a membrane localization sequence.
